# EUROPÄISCHE PATENTSCHRIFT

(11) **EP 1 069 136 B1**
(45) Veröffentlichungstag und Bekanntmachung des Hinweises auf die Patenterteilung: **29.02.2012**
(21) Anmeldenummer: 00114542.4
(22) Anmeldetag: 06.07.2000
(51) Int. Cl.: C07K 14/47, C12P 21/02, C12N 15/12, C12N 1/21

(54) **Verfahren zur rekombinanten Herstellung von Ribonukleoproteinen**
Method for the recombinant production of ribonucleoproteins
Procédé de production recombinante de ribonucléoproteins

(30) Priorität: 07.07.1999 DE 19931380
(43) Veröffentlichungstag der Anmeldung: 17.01.2001
(73) Patentinhaber: F. Hoffmann-La Roche AG, 4070 Basel (CH)
(72) Erfinder: Burckhardt, Jean Dr., 4312 Magden (CH); Haass, Michael Dr., 79359 Neuenburg (DE); Lehmann, Hans-Peter, Dr., 82377 Penzberg (DE)
(74) Vertreter: Weiss, Wolfgang

(56) Entgegenhaltungen:
- DEUTSCHER, S.L. ET AL.: "Molecular analysis of the 60-kDa human Ro ribonucleoprotein" PNAS, Bd. 85, 1988, Seiten 9479-9483, XP000941349
- WANG, D. ET AL.: "Cloning and expression of mouse 60 kDa ribonucleoprotein SS-A/Ro" MOLECULAR BIOLOGY REPORTS, Bd. 23, 1996, Seiten 205-210, XP000942707
- GRANGER, D. ET AL.: "RNA-labelled Ro and La ribonucleoprotein complexes reassembled in vitro; characterization by gel shift analysis" CLIN. EXP. IMMUNOL., Bd. 106, 1996, Seiten 498-503, XP000942733
- FARRIS, A. D. ET AL.: "Conserved features of Y RNAs revealed by automated phylogenetic secondary structure analysis" NUCLEIC ACIDS RESEARCH, Bd. 27, 15. Februar 1999 (1999-02-15), Seiten 1070-1078, XP000941352

### SEQUENZPROTOKOLL

<110> Roche Diagnostics GmbH
<120> Verfahren zur rekombinanten Herscellung von Ribonukleoproteinen
<130> 20657PDE Ribonukleoproteine
<140> 19931380.6
   <141> 1999-07-07
<160> 14
<170> PatentIn Ver. 2.1
<210> 1
   <211> 552
   <212> PRT
   <213> Homo sapiens
<220>
   <223> SSA60 M4-C6
<400> 1
   Met Arg Gly Ser His His His His His His Gly Ser Met Glu Glu Ser
     1               5                  10                  15
   Val Asn Gln Met Gln Pro Leu Asn Glu Lys Gln Ile Ala Asn Ser Gln
                20                  25                  30
   Asp Gly Tyr Val Trp Gln Val Thr Asp Met Asn Arg Leu His Arg Phe
            35                  40                  45
   Leu Cys Phe Gly Ser Glu Gly Gly Thr Tyr Tyr Ile Lys Glu Gln Lys
        50                  55                  60
   Leu Gly Leu Glu Asn Ala Glu Ala Leu Ile Arg Leu Ile Glu Asp Gly
    65                  70                  75                  80
   Arg Gly Cys Glu Val Ile Gln Glu Ile Lys Ser Phe Ser Gln Glu Gly
                    85                  90                  95
   Arg Thr Thr Lys Gln Glu Pro Met Leu Phe Ala Leu Ala Ile Cys Ser
               100                 105                 110
   Gln Cys Ser Asp Ile Ser Thr Lys Gln Ala Ala Phe Lys Ala Val Ser
           115                 120                 125
   Glu Val Cys Arg Ile Pro Thr His Leu Phe Thr Phe Ile Gln Phe Lys
   130 135 140
   Lys Asp Leu Lys Glu Ser Met Lys Cys Gly Met Trp Gly Arg Ala Leu
   145 150 155 160
   Arg Lys Ala Ile Ala Asp Trp Tyr Asn Glu Lys Gly Gly Met Ala Leu
   165 170 175
   Ala Leu Ala Val Thr Lys Tyr Lys Gln Arg Asn Gly Trp Ser His Lys
   180 185 190
   Asp Leu Leu Arg Leu Ser His Leu Lys Pro Ser Ser Glu Gly Leu Ala
   195 200 205
   Ile Val Thr Lys Tyr Ile Thr Lys Gly Trp Lys Glu Val His Glu Leu
   210 215 220
   Tyr Lys Glu Lys Ala Leu Ser Val Glu Thr Glu Lys Leu Leu Lys Tyr
   225 230 235 240
   Leu Glu Ala Val Glu Lys Val Lys Arg Thr Lys Asp Glu Leu Glu Val
   245 250 255
   Ile His Leu Ile Glu Glu His Arg Leu Val Arg Glu His Leu Leu Thr
   260 265 270
   Asn His Leu Lys Ser Lys Glu Val Trp Lys Ala Leu Leu Gln Glu Met
   275 280 285
   Pro Leu Thr Ala Leu Leu Arg Asn Leu Gly Lys Met Thr Ala Asn Ser
   290 295 300
   Val Leu Glu Pro Gly Asn Ser Glu Val Ser Leu Val Cys Glu Lys Leu
   305 310 315 320
   Cys Asn Glu Lys Leu Leu Lys Lys Ala Arg Ile His Pro Phe His Ile
   325 330 335
   Leu Ile Ala Leu Glu Thr Tyr Lys Thr Gly His Gly Leu Arg Gly Lys
   340 345 350
   Leu Lys Trp Arg Pro Asp Glu Glu Ile Leu Lys Ala Leu Asp Ala Ala
   355 360 365
   Phe Tyr Lys Thr Phe Lys Thr Val Glu Pro Thr Gly Lys Arg Phe Leu
   370 375 380
   Leu Ala Val Asp Val Ser Ala Ser Met Asn Gln Arg Val Leu Gly Ser
   385 390 395 400
   Ile Leu Asn Ala Ser Thr Val Ala Ala Ala Met Cys Met Val Val Thr
   405 410 415
   Arg Thr Glu Lys Asp Ser Tyr Val Val Ala Phe Ser Asp Glu Met Val
   420 425 430
   Pro Cys Pro Val Thr Thr Asp Met Thr Leu Gln Gln Val Leu Met Ala
   435 440 445
   Met Ser Gln Ile Pro Ala Gly Gly Thr Asp Cys Ser Leu Pro Met Ile
   450 455 460
   Trp Ala Gln Lys Thr Asn Thr Pro Ala Asp Val Phe Ile Val Phe Thr
   465 470 475 480
   Asp Asn Glu Thr Phe Ala Gly Gly Val His Pro Ala Ile Ala Leu Arg
   485 490 495
   Glu Tyr Arg Lys Lys Met Asp Ile Pro Ala Lys Leu Ile Val Cys Gly
   500 505 510
   Met Thr Ser Asn Gly Phe Thr Ile Ala Asp Pro Asp Asp Arg Gly Met
   515 520 525
   Leu Asp Met Cys Gly Phe Asp Thr Gly Ala Leu Asp Val Ile Arg Asn
   530 535 540
   Phe Thr Leu Asp Met Ile Val Asp
   545 550
<210> 2
   <211> 553
   <212> PRT
   <213> Homo sapiens
<220>
   <223> SSA60 M56
<400> 2
   Met Arg Gly Ser His His His His His His Gly Asp Asp Asp Asp Lys
   1 5 10 15
   Glu Glu Ser Val Asn Gln Met Gln Pro Leu Asn Glu Lys Gln Ile Ala
   20 25 30
   Asn Ser Gln Asp Gly Tyr Val Trp Gln Val Thr Asp Met Asn Arg Leu
   35 40 45
   His Arg Phe Leu Cys Phe Gly Ser Glu Gly Gly Thr Tyr Tyr Ile Lys
   50 55 60
   Glu Gln Lys Leu Gly Leu Glu Asn Ala Glu Ala Leu Ile Arg Leu Ile
   65 70 75 80
   Glu Asp Gly Arg Gly Cys Glu Val Ile Gln Glu Ile Lys Ser Phe Ser
   85 90 95
   Gln Glu Gly Arg Thr Thr Lys Gln Glu Pro Met Leu Phe Ala Leu Ala
   100 105 110
   Ile Cys Ser Gln Cys Ser Asp Ile Ser Thr Lys Gln Ala Ala Phe Lys
   115 120 125
   Ala Val Ser Glu Val Cys Arg Ile Pro Thr His Leu Phe Thr Phe Ile
   130 135 140
   Gln Phe Lys Lys Asp Leu Lys Glu Ser Met Lys Cys Gly Met Trp Gly
   145 150 155 160
   Arg Ala Leu Arg Lys Ala Ile Ala Asp Trp Tyr Asn Glu Lys Gly Gly
   165 170 175
   Met Ala Leu Ala Leu Ala Val Thr Lys Tyr Lys Gln Arg Asn Gly Trp
   180 185 190
   Ser His Lys Asp Leu Leu Arg Leu Ser His Leu Lys Pro Ser Ser Glu
   195 200 205
   Gly Leu Ala Ile Val Thr Lys Tyr Ile Thr Lys Gly Trp Lys Glu Val
   210 215 220
   His Glu Leu Tyr Lys Glu Lys Ala Leu Ser Val Glu Thr Glu Lys Leu
   225 230 235 240
   Leu Lys Tyr Leu Glu Ala Val Glu Lys Val Lys Arg Thr Lys Asp Glu
   245 250 255
   Leu Glu Val Ile His Leu Ile Glu Glu His Arg Leu Val Arg Glu His
   260 265 270
   Leu Leu Thr Asn His Leu Lys Ser Lys Glu Val Trp Lys Ala Leu Leu
   275 280 285
   Gln Glu Met Pro Leu Thr Ala Leu Leu Arg Asn Leu Gly Lys Met Thr
   290 295 300
   Ala Asn Ser Val Leu Glu Pro Gly Asn Ser Glu Val Ser Leu Val Cys
   305 310 315 320
   Glu Lys Leu Cys Asn Glu Lys Leu Leu Lys Lys Ala Arg Ile His Pro
   325 330 335
   Phe His Ile Leu Ile Ala Leu Glu Thr Tyr Lys Thr Gly His Gly Leu
   340 345 350
   Arg Gly Lys Leu Lys Trp Arg Pro Asp Glu Glu Ile Leu Lys Ala Leu
   355 360 365
   Asp Ala Ala Phe Tyr Lys Thr Phe Lys Thr Val Glu Pro Thr Gly Lys
   370 375 380
   Arg Phe Leu Leu Ala Val Asp Val Ser Ala Ser Met Asn Gln Arg Val
   385 390 395 400
   Leu Gly Ser Ile Leu Asn Ala Ser Thr Val Ala Ala Ala Met Cys Met
   405 410 415
   Val Val Thr Arg Thr Glu Lys Asp Ser Tyr Val Val Ala Phe Ser Asp
   420 425 430
   Glu Met Val Pro Cys Pro Val Thr Thr Asp Met Thr Leu Gln Gln Val
   435 440 445
   Leu Met Ala Met Ser Gln Ile Pro Ala Gly Gly Thr Asp Cys Ser Leu
   450 455 460
   Pro Met Ile Trp Ala Gln Lys Thr Asn Thr Pro Ala Asp Val Phe Ile
   465 470 475 480
   Val Phe Thr Asp Asn Glu Thr Phe Ala Gly Gly Val His Pro Ala Ile
   485 490 495
   Ala Leu Arg Glu Tyr Arg Lys Lys Met Asp Ile Pro Ala Lys Leu Ile
   500 505 510
   Val Cys Gly Met Thr Ser Asn Gly Phe Thr Ile Ala Asp Pro Asp Asp
   515 520 525
   Arg Gly Met Leu Asp Met Cys Gly Phe Asp Thr Gly Ala Leu Asp Val
   530 535 540
   Ile Arg Asn Phe Thr Leu Asp Met Ile
   545 550
<210> 3
   <211> 101
   <212> DNA
   <213> Homo sapiens
<220>
   <223> HY3 (Wolin et al., PNAS 81 (1984), 1996-2000)
<400> 3
   ggctggtccg agtgcagtgg tgtttacaac taattgatca caaccagtta cagatttctt 60
   tgttccttct ccactcccac tgcttcactt gactagcctt t 101
<210> 4
   <211> 150
   <212> DNA
   <213> Homo sapiens
<220>
   <223> HY3 aus SSA60 M56
<400> 4
   tttatttgct ttgtgagcgg ataacaatta taatagattc aggctggtcc gagtgcagtg 60
   gtgtttacaa ctaattgatc acaaccagtt acagatttct ttgttccttc ttcactccca 120
   ctgcttcact tgactagcct ttgccgccag 150
<210> 5
   <211> 2220
   <212> DNA
   <213> Homo sapiens
<220>
   <221> CDS
   <222> (533)..(2191)
<400> 5
   gcgacacgga aatgttgaat actcatactc ttcctttttc aatattattg aagcatttat 60
   cagggttatt gtctcatgag cggatacata tttgaatgta tttagaaaaa taaacaaata 120
   ggggttccgc gcacatttcc ccgaaaagtg ccacctgacg tctaagaaac cattattatc 180
   atgacattaa cctataaaaa taggcgtatc acgaggccct ttcgtcttca cctcgagaaa 240
   tcataaaaaa tttatttgct ttgtgagcgg ataacaatta taatagattc aggctggtcc 300
   gagtgcagtg gtgtttacaa ctaattgatc acaaccagtt acagatttct ttgttccttc 360
   ttcactccca ctgcttcact tgactagcct ttgccgccag ttccgctggc ggcattttct 420
   cgagaaatca taaaaaattt atttgctttg tgagcggata acaattataa tagattcaat 480
   tgtgagcgga taacaatttc acacagaatt cattaaagag gagaaattaa cc atg gga 538
                                                            Met Gly
                                                             1
   gga tcc cat cac cat cac cat cac ggt gat gac gat gac aaa gag gaa    586
   Gly Ser His His His His His His Gly Asp Asp Asp Asp Lys Glu Glu
               5                  10                  15
   tct gta aac caa atg cag cca ctg aat gag aag cag ata gcc aat tct    634
   Ser Val Asn Gln Met Gln Pro Leu Asn Glu Lys Gln Ile Ala Asn Ser
           20                  25                  30
   cag gat gga tat gta tgg caa gtc act gac atg aat cga cta cac cgg    682
   Gln Asp Gly Tyr Val Trp Gln Val Thr Asp Met Asn Arg Leu His Arg
       35                  40                  45                  50
   ttc tta tgt ttc ggt tct gaa ggt ggg act tat tat atc aaa gaa cag    730
   Phe Leu Cys Phe Gly Ser Glu Gly Gly Thr Tyr Tyr Ile Lys Glu Gln
                   55                  60                  65
   aag ttg ggc ctt gaa aat gct gaa gct tta att aga ttg att gaa gat    778
   Lys Leu Gly Leu Glu Asn Ala Glu Ala Leu Ile Arg Leu Ile Glu Asp
               70                  75                  80
   ggc aga gga tgt gaa gtg ata caa gaa ata aag tca ttt agt caa gaa    826
   Gly Arg Gly Cys Glu Val Ile Gln Glu Ile Lys Ser Phe Ser Gln Glu
           85                  90                  95
   ggc aga acc aca aag caa gag cct atg ctc ttt gca ctt gcc att tgt    874
   Gly Arg Thr Thr Lys Gln Glu Pro Met Leu Phe Ala Leu Ala Ile Cys
       100                 105                 110
   tcc cag tgc tcc gat atc agc aca aaa caa gca gca ttc aag gct gtt    922
   Ser Gln Cys Ser Asp Ile Ser Thr Lys Gln Ala Ala Phe Lys Ala Val
   115                 120                 125                 130
   tct gaa gtt tgt cgc att cct acc cat ctc ttt act ttt atc cag ttt    970
   Ser Glu Val Cys Arg Ile Pro Thr His Leu Phe Thr Phe Ile Gln Phe
                   135                 140                 145
   aag aaa gac ctg aag gaa agc atg aaa tgt ggc atg tgg ggt cgt gcc   1018
   Lys Lys Asp Leu Lys Glu Ser Met Lys Cys Gly Met Trp Gly Arg Ala
               150                 155                 160
   ctc cgg aag gct ata gcg gac tgg tac aat gag aaa ggt ggc atg gcc   1066
   Leu Arg Lys Ala Ile Ala Asp Trp Tyr Asn Glu Lys Gly Gly Met Ala
           165                 170                 175
   ctt gct ctg gca gtt aca aaa tat aaa cag aga aat ggc tgg tct cac   1114
   Leu Ala Leu Ala Val Thr Lys Tyr Lys Gln Arg Asn Gly Trp Ser His
       180                 185                 190
   aaa gat cta tta aga ttg tca cat ctt aaa cct tcc agt gaa gga ctt   1162
   Lys Asp Leu Leu Arg Leu Ser His Leu Lys Pro Ser Ser Glu Gly Leu
   195                 200                 205                 210
   gca att gtg acc aaa tat att aca aag ggc tgg aaa gaa gtt cat gaa   1210
   Ala Ile Val Thr Lys Tyr Ile Thr Lys Gly Trp Lys Glu Val His Glu
                   215                 220                 225
   ttg tat aaa gaa aaa gca ctc tct gtg gag act gaa aaa tta tta aag   1258
   Leu Tyr Lys Glu Lys Ala Leu Ser Val Glu Thr Glu Lys Leu Leu Lys
               230                 235                 240
   tat ctg gag gct gta gag aaa gtg aag cgc aca aaa gat gag cta gaa   1306
   Tyr Leu Glu Ala Val Glu Lys Val Lys Arg Thr Lys Asp Glu Leu Glu
           245                 250                 255
   gtc att cat cta ata gaa gaa cat aga tta gtt aga gaa cat ctt tta   1354
   Val Ile His Leu Ile Glu Glu His Arg Leu Val Arg Glu His Leu Leu
       260                 265                 270
   aca aat cac tta aag tct aaa gag gta tgg aag gct ttg tta caa gaa   1402
   Thr Asn His Leu Lys Ser Lys Glu Val Trp Lys Ala Leu Leu Gln Glu
   275                 280                 285                 290
   atg ccg ctt act gca tta cta agg aat cta gga aag atg act gct aat   1450
   Met Pro Leu Thr Ala Leu Leu Arg Asn Leu Gly Lys Met Thr Ala Asn
                   295                 300                 305
   tca gta ctt gaa cca gga aat tca gaa gta tct tta gta tgt gaa aaa   1498
   Ser Val Leu Glu Pro Gly Asn Ser Glu Val Ser Leu Val Cys Glu Lys
               310                 315                 320
   ctg tgt aat gaa aaa cta tta aaa aag gct cgt ata cat cca ttt cat    1546
   Leu Cys Asn Glu Lys Leu Leu Lys Lys Ala Arg Ile His Pro Phe His
           325                 330                 335
   att ttg atc gca tta gaa act tac aag aca ggt cat ggt ctc aga ggg    1594
   Ile Leu Ile Ala Leu Glu Thr Tyr Lys Thr Gly His Gly Leu Arg Gly
       340                 345                 350
   aaa ctg aag tgg cgc cct gat gaa gaa att ttg aaa gca ttg gat gct    1642
   Lys Leu Lys Trp Arg Pro Asp Glu Glu Ile Leu Lys Ala Leu Asp Ala
   355                 360                 365                 370
   gct ttt tat aaa aca ttt aag aca gtt gaa cca act gga aaa cgt ttc    1690
   Ala Phe Tyr Lys Thr Phe Lys Thr Val Glu Pro Thr Gly Lys Arg Phe
                   375                 380                 385
   tta cta gct gtt gat gtc agt gct tct atg aac caa aga gtt ttg ggt    1738
   Leu Leu Ala Val Asp Val Ser Ala Ser Met Asn Gln Arg Val Leu Gly
               390                 395                 400
   agt ata ctc aac gct agt aca gtt gct gca gca atg tgc atg gtt gtc    1786
   Ser Ile Leu Asn Ala Ser Thr Val Ala Ala Ala Met Cys Met Val Val
           405                 410                 415
   aca cga aca gaa aaa gat tct tat gta gtt gct ttt tcc gat gaa atg    1834
   Thr Arg Thr Glu Lys Asp Ser Tyr Val Val Ala Phe Ser Asp Glu Met
       420                 425                 430
   gta cca tgt cca gtg act aca gat atg acc tta caa cag gtt tta atg    1882
   Val Pro Cys Pro Val Thr Thr Asp Met Thr Leu Gln Gln Val Leu Met
   435                 440                 445                 450
   gct atg agt cag atc cca gcg ggt gga act gat tgc tct ctt cca atg    1930
   Ala Met Ser Gln Ile Pro Ala Gly Gly Thr Asp Cys Ser Leu Pro Met
                   455                 460                 465
   atc tgg gct cag aag aca aac aca cct gct gat gtc ttc att gta ttc    1978
   Ile Trp Ala Gln Lys Thr Asn Thr Pro Ala Asp Val Phe Ile Val Phe
               470                 475                 480
   act gat aat gag acc ttt gct gga ggt gtc cat cct gct att gct ctg    2026
   Thr Asp Asn Glu Thr Phe Ala Gly Gly Val His Pro Ala Ile Ala Leu
           485                 490                 495
   agg gag tat cga aag aaa atg gat att cca gct aaa ttg att gtt tgt    2074
   Arg Glu Tyr Arg Lys Lys Met Asp Ile Pro Ala Lys Leu Ile Val Cys
       500                 505                 510
   gga atg aca tca aat ggt ttc acc att gca gac cca gat gat aga ggc    2122
   Gly Met Thr Ser Asn Gly Phe Thr Ile Ala Asp Pro Asp Asp Arg Gly
   515                 520                 525                 530
   atg ttg gat atg tgc ggc ttt gat act gga gct ctg gat gta att cga    2170
   Met Leu Asp Met Cys Gly Phe Asp Thr Gly Ala Leu Asp Val Ile Arg
                   535                 540                 545
   aat ttc aca tta gat atg att taatagtcga gcttaattag ctgagcttg        2220
   Asn Phe Thr Leu Asp Met Ile
               550
<210> 6
   <211> 553
   <212> PRT
   <213> Homo sapiens
<400> 6
   Met Gly Gly Ser His His His His His His Gly Asp Asp Asp Asp Lys
   1               5                   10                  15
   Glu Glu Ser Val Asn Gln Met Gln Pro Leu Asn Glu Lys Gln Ile Ala
               20                  25                  30
   Asn Ser Gln Asp Gly Tyr Val Trp Gln Val Thr Asp Met Asn Arg Leu
           35                  40                  45
   His Arg Phe Leu Cys Phe Gly Ser Glu Gly Gly Thr Tyr Tyr Ile Lys
       50                  55                  60
   Glu Gln Lys Leu Gly Leu Glu Asn Ala Glu Ala Leu Ile Arg Leu Ile
   65                  70                  75                  80
   Glu Asp Gly Arg Gly Cys Glu Val Ile Gln Glu Ile Lys Ser Phe Ser
                   85                  90                  95
   Gln Glu Gly Arg Thr Thr Lys Gln Glu Pro Met Leu Phe Ala Leu Ala
               100                 105                 110
   Ile Cys Ser Gln Cys Ser Asp Ile Ser Thr Lys Gln Ala Ala Phe Lys
           115                 120                 125
   Ala Val Ser Glu Val Cys Arg Ile Pro Thr His Leu Phe Thr Phe Ile
       130                 135                 140
   Gln Phe Lys Lys Asp Leu Lys Glu Ser Met Lys Cys Gly Met Trp Gly
   145 150 155 160
   Arg Ala Leu Arg Lys Ala Ile Ala Asp Trp Tyr Asn Glu Lys Gly Gly
   165 170 175
   Met Ala Leu Ala Leu Ala Val Thr Lys Tyr Lys Gln Arg Asn Gly Trp
   180 185 190
   Ser His Lys Asp Leu Leu Arg Leu Ser His Leu Lys Pro Ser Ser Glu
   195 200 205
   Gly Leu Ala Ile Val Thr Lys Tyr Ile Thr Lys Gly Trp Lys Glu Val
   210 215 220
   His Glu Leu Tyr Lys Glu Lys Ala Leu Ser Val Glu Thr Glu Lys Leu
   225 230 235 240
   Leu Lys Tyr Leu Glu Ala Val Glu Lys Val Lys Arg Thr Lys Asp Glu
   245 250 255
   Leu Glu Val Ile His Leu Ile Glu Glu His Arg Leu Val Arg Glu His
   260 265 270
   Leu Leu Thr Asn His Leu Lys Ser Lys Glu Val Trp Lys Ala Leu Leu
   275 280 285
   Gln Glu Met Pro Leu Thr Ala Leu Leu Arg Asn Leu Gly Lys Met Thr
   290 295 300
   Ala Asn Ser Val Leu Glu Pro Gly Asn Ser Glu Val Ser Leu Val Cys
   305 310 315 320
   Glu Lys Leu Cys Asn Glu Lys Leu Leu Lys Lys Ala Arg Ile His Pro
   325 330 335
   Phe His Ile Leu Ile Ala Leu Glu Thr Tyr Lys Thr Gly His Gly Leu
   340 345 350
   Arg Gly Lys Leu Lys Trp Arg Pro Asp Glu Glu Ile Leu Lys Ala Leu
   355 360 365
   Asp Ala Ala Phe Tyr Lys Thr Phe Lys Thr Val Glu Pro Thr Gly Lys
   370 375 380
   Arg Phe Leu Leu Ala Val Asp Val Ser Ala Ser Met Asn Gln Arg Val
   385 390 395 400
   Leu Gly Ser Ile Leu Asn Ala Ser Thr Val Ala Ala Ala Met Cys Met
   405 410 415
   Val Val Thr Arg Thr Glu Lys Asp Ser Tyr Val Val Ala Phe Ser Asp
   420 425 430
   Glu Met Val Pro Cys Pro Val Thr Thr Asp Met Thr Leu Gln Gln Val
   435 440 445
   Leu Met Ala Met Ser Gln Ile Pro Ala Gly Gly Thr Asp Cys Ser Leu
   450 455 460
   Pro Met Ile Trp Ala Gln Lys Thr Asn Thr Pro Ala Asp Val Phe Ile
   465 470 475 480
   Val Phe Thr Asp Asn Glu Thr Phe Ala Gly Gly Val His Pro Ala Ile
   485 490 495
   Ala Leu Arg Glu Tyr Arg Lys Lys Met Asp Ile Pro Ala Lys Leu Ile
   500 505 510
   Val Cys Gly Met Thr Ser Asn Gly Phe Thr Ile Ala Asp Pro Asp Asp
   515 520 525
   Arg Gly Met Leu Asp Met Cys Gly Phe Asp Thr Gly Ala Leu Asp Val
   530 535 540
   Ile Arg Asn Phe Thr Leu Asp Met Ile
   545 550
<210> 7
   <211> 84
   <212> DNA -
   <213> Künstliche Sequenz
<220>
   <223> Beschreibung der künstlichen Sequenz:Primer HY3-SynF
<400> 7
   acttggtacc gaaattaata cgactcacta tagggagagg ctggtccgag tgcagtggtg 60
   tttacaacta attgatcaca acca 84
<210> 8
   <211> 85
   <212> DNA
   <213> Künstliche Sequenz
<220>
   <223> Beschreibung der künstlichen Sequenz:Primer HY3-SynR
<400> 8
   gtgtctcgag aaaggctagt caagtgaagc agtgggagtg gacaaggaac aaagaaatct 60
   gtaactggtt gtgatcaatt agttg 85
<210> 9
   <211> 89
   <212> DNA
   <213> Künstliche Sequenz
<220>
   <223> Beschreibung der künstlichen Sequenz:Primer SSA60M6-NF
<400> 9
   cacagaattc attaaagagg agaaattaac tatgagagga tcccatcacc atcaccatca 60
   cggtgatgac gatgacaaag aggaatctg 89
<210> 10
   <211> 27
   <212> DNA
   <213> Künstliche Sequenz
<220>
   <223> Beschreibung der künstliche Sequenz:Primer SSA60M6-NRev
<400> 10
   ctaattaaag cttcagcact ttcaagg 27
<210> 11
   <211> 68
   <212> DNA
   <213> Künstliche Sequenz
<220>
   <223> Beschreibung der künstlichen Sequenz:Primer SSA60M5-CF
<400> 11
   gatactggag ctctggatgt aattcgaaat ttcacattag atatgattta atagtcgcga 60
   gccagctt 68
<210> 12
   <211> 28
   <212> DNA
   <213> Künstliche Sequenz
<220>
   <223> Beschreibung der künstliche Sequenz:Primer SSA60MS-CRev
<400> 12
   aggcagctct agagcggcgg atttgtcc 28
<210> 13
   <211> 82
   <212> DNA
   <213> Künstliche Sequenz
<220>
   <223> Beschreibung der künstlichen Sequenz:Primer HYFPQE
<400> 13
   acttctcgag aaatcataaa aaatttattt gctttgtgag cggataacaa ttataataga 60
   ttcaggctgg tccgagtgca gt                                          82
<210> 14
   <211> 62
   <212> DNA
   <213> Künstliche Sequenz
<220>
   <223> Beschreibung der künstlichen Sequenz:Primer HYRPQE
<400> 14
   gtgtctcgag aaaatgccgc cagcggaact ggcggcaaag gctagtcaag tgaagcagtg 60
   gg                                                                62

## Beschreibung

Die Erfindung betrifft ein Verfahren zur rekombinanten Herstellung eines Ribonukleoproteins in prokaryontischen Zellen.

Im Serum von Patienten mit der Autoimmunerkrankung SLE (systemischer Lupus Erythematosus) treten häufig Antikörper auf, die gegen Ribonukleoproteine, wie etwa das SSA60-Autoantigen, gerichtet sind. Das SSA60-Antigen ist ein RNA-bindendes Molekül, das im Cytoplasma und im Nukleus verschiedener Zelltypen vorkommt. Das SSA60-Antigen ist häufig an eine HY-RNA gebunden, wie z.B. HY1, HY3, HY4 oder HY5, die jeweils etwa 100 Basen lang sind und eine sehr ähnliche Struktur aufweisen. HY-RNA-Moleküle, die in vielen eukaryontischen Organismen nachgewiesen werden konnten, kommen in Prokaryonten nicht vor.

Eine Analyse der Antikörperantwort auf Ribonukleoproteine ist von großem Interesse, um eine Diagnose von Autoimmunerkrankungen, insbesondere SLE, aufstellen zu können, da die Gegenwart von gegen Autoantigene gerichteten Antikörpern eine bestehende Autoimmunerkrankung anzeigt und/oder eine Prognose für ein mögliches zukünftiges Auftreten einer Autoimmunerkrankung erlaubt.

Das SSA60-Antigen wird häufig auch als SSA/Ro bezeichnet. Es handelt sich um ein Protein mit einer Größe von etwa 60 kD. Davon ist sowohl hinsichtlich der Sequenz als auch der Funktion das SSA52-Antigen zu unterscheiden, wobei jedoch unter bestimmten Bedingungen in vivo eine Assoziation des SSA52-Proteins mit dem SSA60-Protein auftreten kann.

Die Sequenz der cDNA von SSA60 wurde von Deutscher et al., Proc. Natl. Acd. Sci. USA, Vol. 85 (1988), 9479-9483 veröffentlicht. Dieses Dokument beschreibt die molekulare Analyse des 60-kDa humanen Ro-Ribonukleoproteins, wobei rekombinantes Ro-Protein und humane Y1-RNA in vitro rekonstituiert wurden. HY-RNAs wurden von Hendrick et al., J. Mol. Biol. 1 (1981), 1138-1149 sowie von Wolin et al., Proc. Natl. Acad. Sci. USA 81 (1984), 1996-2000 beschrieben

Wang et al. (Molecular Biology Reports, Vol. 23, 205-210 (1996)) beschreiben die Klonierung und Expression von murinem 60-kDa-Ribonukleoprotein SSA/Ro.

Granger et al. (Clin. Exp. Immunol., Vol., 106 498-503 (1996)) offenbaren RNA-markierte Ro- und La-Ribonukleoproteinkomplexe, die in vitro reassembliert wurden, sowie ihre Charakterisierung mittels Gelshift-Assays. Als RNA wird in vitro transkribierte und markierte HY5-RNA verwendet, die Proteinkomponenten stammen aus HeLa-Zellextrakten.

Ferris et al. (Nucleic Acids Research, Vol. 27, 1070-1078 (1999)) beschreiben die Entdeckung konservierter Eigenschaften von Y-RNAs durch automatisierte phylogenetische Sekundärstrukturanalyse, wobei ein bestimmter Algorithmus eingesetzt wird. Y-RNA-Reinigung durch Immunopräzipitation wird ebenfalls erwähnt.

Bisher wurden für diagnostische Verfahren, beispielsweise unter Verwendung eines EIA (Enzymimmunoassy)-Verfahrens natives gereinigtes SSA60-Antigen (z.B. Rindermilzantigen von Immunovision) sowie reine rekombinante SSA60-Antigene aus Baculovirus oder E.coli ohne RNA eingesetzt. Dabei ist die dreidimensionale Struktur (Faltung) des Antigens von essentieller Bedeutung für die immunologische Erkennung aller relevanten Patientenseren. Die Konformation und Reproduzierbarkeit des SSA60-Antigens variiert je nach Herstellungsverfahren wie folgt:
a) Nativ gereinigtes Protein ist der "Goldstandard" bezüglich Sensitivität für SSA60 Autoantikörper (z.B. Immunovision). Dieses Material repräsentiert die physiologische (native) Konformation. Bei Einsatz dieses Antigen können aber auch falsch-positive Resultate auftreten, z.B. durch die Erkennung natürlicher Autoantikörper, die nicht krankheitsrelevant sind. Desweiteren hängt die Reproduzierbarkeit der Aufreinigung von der Selektion der Immunaffinitätsmatrix ab.
b) Rekombinantes Protein aus E.coli liegt im Wesentlichen in denaturierter, also linearer Konformation vor, da in E.coli keine posttranslationale Modifikation von Proteinen erfolgt. Dadurch wird ein bestimmter Anteil von Patientenseren nicht erfaßt. Allerdings ist die Detektion krankheitsrelevanter Autoantikörper spezifischer im Vergleich zu nativem Antigen. Mit dieser Methode können eine gute Reproduzierbarkeit und Ausbeute erhalten werden. Bei dieser Testführung wird rekombinantes freies SSA60-Antigen aus E.coli eingesetzt, das nicht an RNA assoziiert ist. Dazu wird z.B. die cDNA von SSA60 mit Primern, die gemäß der SSA60-Sequenz von Deutscher et al., supra, hergestellt werden, aus einer cDNA-Bank gefischt, kloniert und sequenziert. C.H.A. Veldhofen et al., J. Immunol. Methods 151 (1992) 177-189 beschreibt die Entwicklung eines quantitativen Assays für die Detektion von Antikörpern gegen SSA60 (RO/SSA) unter Verwendung von rekombinanten freien Proteinen, die in E.coli kloniert und exprimiert wurden. Es zeigte sich aber, daß einige Seren, die mit anderen Tests als SSA60 positive Seren identifiziert werden können, mit solchen Antigen-Präparationen ohne RNA nicht detektiert werden können, da die Antigene in einer denaturierten Form vorliegen (G. Boire et al., Arthrithis and Rheumatism, Vol. 34 No. 6 (1991), 722-730; B. William St. Clair et al., Arthritis and Rheumatism, Vol. 37 No. 9 (1994), 1373-1379).
c) Rekombinantes Protein aus Baculovirus kann als "pseudo-natives" SSA60-Antigen betrachtet werden, da in diesem eukaryontschen Expressionssystem die Faltung bei der Proteinbiosynthese (zumindest die dafür notwendige Disulfidverbrückung) korrekt erfolgt. Die immunologische Reaktivität ist mit derjenigen des nativ isolierten SSA60-Antigens vergleichbar. Das Problem bei der Herstellung ist die schlechte Ausbeute und die kostenintensive Zellkultur. Es werden zwar gute Testergebnisse erzielt, die Gewinnung von großen Mengen an Autoantigenen für kommerzielle Diagnoseverfahren ist bei dieser Verfahrensführung jedoch aufwendig und mit den bei der Handhabung einer Zellkultur auftretenden Problemen verbunden.

Es war deshalb eine Aufgabe der Erfindung ein einfach durchzuführendes Verfahren bereitzustellen, mit dem Ribonukloproteine in einer Form erhalten werden, die eine hohe Selektivität und Testsicherheit bei diagnostischen Verfahren liefert.

Diese Aufgabe wird erfindungsgemäß gelöst durch ein Verfahren zur rekombinanten Herstellung eines SSA-60-Ribonukleoproteins oder eines Derivates davon, welches die Schritte umfaßt:
(a) Bereitstellen einer prokaryontischen Wirtszelle, die (i) mindestens eine für eine Ribonukleinsäurekomponente des SSA60-Ribonukleoproteins codierende DNA und (ii) mindestens eine für eine Proteinkomponente des SSA60-Ribonukleoproteins codierende DNA enthält,
(b) Exprimieren der DNAs (i) und (ii) unter Bedingungen, bei denen ein Ribonukleoprotein gebildet wird und
(c) Gewinnen des Ribonukleoproteins.

Überraschenderweise wurde festgestellt, daß in prokaryontischen Zellen Ribonukleoproteine durch parallele Expression der Ribonukleinsäure- und der Proteinkomponente rekombinant in funktioneller, z.B. immunologisch aktiver Form, hergestellt werden können. Auf diese Weise ist eine einfache und kostengünstige Herstellung von Ribonukleoproteinen im großen Maßstab möglich. Bevorzugt werden die Protein- und die Ribonukleinsäurekomponente in einer assoziierten Form erhalten. Die Ribonukleoproteine können in ihrer nativen und somit zumeist löslichen Form hergestellt werden. Es hat sich gezeigt, daß Seren von Patienten mit SLE, die in Tests mit rekombinant hergestellten Proteinen ohne Ribonukleinsäurekomponente negativ sind, mit den erfindungsgemäß hergestellten Ribonukleoproteinen nachgewiesen werden konnten.

Bei dem erfindungsgemäßen Verfahren handelt es sich um ein gut reproduzierbares und kostengünstiges Verfahren, mit dem ein SSA60-Antigen mit guter immunologischer Reaktivität zur Detektion krankheitsrelevanter SSA60-Autoantikörper hergestellt werden kann.

Die Ribonukleoproteine können aus den prokaryontischen Zellen oder/und aus dem zur Kultivierung der Zellen verwendeten Medium gewonnen werden. Es werden bevorzugt gram-negative Zellen und insbesondere E.coli Zellen verwendet.

Die für die Ribonukleinsäurekomponente codierende DNA und die für die Proteinkomponente codierende DNA können in die prokaryontische Wirtszelle auf einem DNA-Konstrukt eingebracht werden. Es ist aber auch möglich, die für die einzelnen Komponenten codierenden DNAs auf getrennten DNA-Konstrukten einzubringen. Mit dem erfindungsgemäßen Verfahren können SSA60-Ribonukleoproteine hergestellt werden, wobei geeigneterweise die Proteinkomponente(n) und die entsprechende(n) zugehörige(n) Nukleinsäurekomponente(n) in einer Wirtszelle exprimiert werden.

Bevorzugt stellt man ein Eukaryonten- insbesondere ein Säuger- und besonders bevorzugt ein humanes Ribonukleoprotein oder ein Derivat davon her. Ein Derivat ist gegenüber der nativen Form in der Sequenz der Ribonukleinsäure und/oder der Sequenz des Proteins modifiziert, beispielsweise durch Substitution, Deletion, Insertion oder/und Addition von einzelnen oder mehreren Aminosäuren bzw. Nucleobasen, wobei jedoch die Fähigkeit der Komponenten zur Assoziierung zum Ribonukleoprotein erhalten bleibt.

Es wird ein SSA60-Ribonukleoprotein exprimiert. Das humane SSA60-Antigen ist ein Protein mit 525 (Form a) bzw. 538 (Form b) Aminosäuren (vgl. Deutscher et al., supra). Ein unterschiedliches Splicing des gleichen primären Transkripts führt zu den zwei verschiedenen Formen des SSA60-Proteins. Die zwei Proteine unterscheiden sich nur von der Aminosäure 515 an, wobei die Aminosäuren 1 bis 514 konstant sind. Bevorzugt wird mit dem erfindungsgemäßen Verfahren ein SSA60-Protein exprimiert, das den konstanten Bereich der Aminosäuren 1 bis 514 umfaßt, sowie SSA60-Proteinderivate, die gegenüber der nativen Form in der Sequenz modifiziert sind, wobei jedoch die Antigen-Epitopeigenschaften beibehalten werden.

Bei der Ribonukleinsäurekomponente handelt es sich bevorzugt um eine HY-RNA, insbesondere um HY1, HY3, HY4 oder/und HY5, am meisten bevorzugt um HY3. Die HY-RNA-Moleküle sind hoch konservierte Moleküle mit einer sehr ähnlichen sekundären Struktur. Sie werden in vivo durch Polymerase III transkribiert und haben eine Größe zwischen 84 und 112 Nukleotiden.

Cytoplasmische RNA, insbesondere menschliche RNA, bevorzugt hY-RNA, liefert einen wichtigen Beitrag zur Ausprägung der nativen Konformation des SSA60-Antigens. Physiologisch liegt das SSA60-Antigen als ein mit SSB, SSA52 und hY-RNA vergesellschaftetes Ribo-Nukleo-Partikel (RNP) vor.

Ein weiterer Aspekt der Erfindung betrifft ein Nukleinsäurekonstrukt umfassend einen Abschnitt, der eine für eine SSA60-Proteinkomponente codierende DNA enthält und einen Abschnitt, der eine für eine Ribonukleinsäurekomponente des SSA60-Ribonukleoproteins codierende DNA enthält. Die codierenden Abschnitte befinden sich bevorzugt in operativer Verknüpfung mit einer Sequenz, die die Expression der Komponenten in prokaryontischen Zellen ermöglicht. Das Konstrukt umfaßt einen Abschnitt, der einen für ein SSA60-Protein oder ein Derivat davon codierenden Abschnitt umfaßt. In einer bevorzugten Ausführungsform umfaßt das Konstrukt einen für HY-RNA codierenden Abschnitt. Besonders bevorzugt ist ein Konstrukt, das einen für SSA60 und einen für HY3 codierenden Abschnitt enthält. Bei Expression eines solchen Konstrukts wird ein assoziiertes Ribonukleoprotein mit der gewünschten immunologisch reaktiven Konvormation des Antigens gebildet. Die Assoziation kann dadurch unterstützt werden, daß eine gleichzeitige Induzierung der Expression von Ribonukleinsäurekomponente und Proteinkomponente erfolgt. Dies kann dadurch erreicht werden, daß sowohl das Gen für die Ribonukleinsäurekomponente als auch das Gen für die Proteinkomponente unter die Kontrolle von gleichen regulierbaren Expressionsystemen, z.B. von lac-Expressionssystemen, gestellt werden.

Ein weiterer Aspekt der Erfindung betrifft eine rekombinante prokaryontische Zelle, die (i) mindestens eine für eine SSA60-Ribonukleinsäurekomponente des SSA60-Ribonukleoproteins codierende DNA enthält und (ii) mindestens eine für eine Proteinkomponente des Ribonukleoproteins codierende DNA enthält. Die codierenden Bereiche können dabei innerhalb der Zelle auf einem einzigen Konstrukt vorliegen oder aber auch getrennt auf mehreren Konstrukten. Die codierenden Bereiche können extrachromosomal, z.B. auf Plasmiden oder/und chromosomal angeordnet sein.

Ein weiterer Gegenstand der Erfindung ist ein rekombinantes Ribonukleoprotein, das durch das oben beschriebene Verfahren erhältlich ist. Durch die Coexpression von Protein und Ribonukleinsäure in prokaryontischen Zellen wird ein Ribonukleoprotein gebildet, welches nicht glykosyliert ist und sich dadurch von in eukaryontischen Wirtszellen gebildeten Ribonukleoproteinen unterscheidet. Bevorzugt umfaßt die Proteinkomponente des rekombinanten Ribonukleoproteins heterologe Hilfssequenzen, die die Expression verbessern oder/und die Aufreinigung erleichtern. Diese Hilfssequenzen können gegebenenfalls Protease-Spaltsequenzen enthalten, so daß sie nach Gewinnung des Produkts entfernt werden können. Ein Beispiel für eine Hilfssequenz ist ein mehrere His-Reste umfassender Sequenzabschnitt (His-Tag).

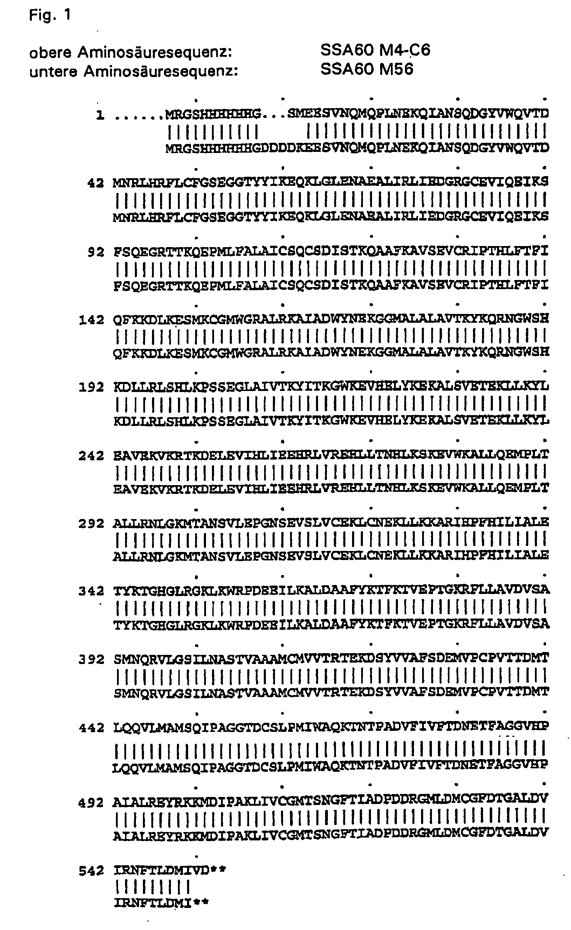

Ein weiterer Gegenstand der Erfindung ist ein SSA60-Protein mit der in Figur 1 gezeigten Sequenz SSA60M56. Das erfindungsgemäße SSA60-Protein weist am N-Terminus eine Hilfssequenz auf, die neben 6 His-Resten eine Spaltsequenz DDDK für das proteolytische Enzym Bovine Enterokinase enthält.

Die Erfindung betrifft weiterhin die Verwendung von rekombinanten SSA60-Ribonukleoproteinen aus Prokaryonten für diagnostische Verfahren. Die Bildung von Ribonukleoproteinen in nativer, insbesondere in assoziierter Form ermöglicht die Bereitstellung von Antigenen, mit denen eine Verbesserung von diagnostischen Verfahren erzielt werden kann. Es wird ein rekombinantes SSA60-Ribonukleoprotein oder ein Derivat davon, insbesondere in Verbindung mit HY3, für die Diagnostik von Autoimmunerkrankungen , z.B. SLE oder Sjogren Syndrom Typ A, eingesetzt.

Ein weiterer Aspekt der Erfindung ist ein Verfahren zum Nachweis eines Analyten in einer Probe unter Verwendung eines Analyt-spezifischen Rezeptors, welches dadurch gekennzeichnet ist, daß man als Rezeptor ein Ribonukleoprotein, wie es hierin beschrieben wird, einsetzt. Bei dem Analyten handelt es sich bevorzugt um einen Antikörper gegen ein Ribonukleoprotein, beispielsweise einen Autoantikörper, wie er bei Autoimmunkrankheiten auftritt. Eine geeignete Testvorgehensweise umfaßt die Schritte
(a) Bereitstellen einer Festphase, die mit einem Ribonukleoprotein beschichtet ist,
(b) Inkontaktbringen der beschichteten Festphase mit einer Probe und
(c) Nachweis einer Bindung zwischen dem Analyten und der beschichteten Festphase.

Als Festphase finden insbesondere Beads Verwendung, es können jedoch auch andere Festphasen, z.B. Oberflächen von Reaktionsgefäßen oder Biochips eingesetzt werden, Die Festphase kann ausschließlich mit dem Ribonukleoprotein oder zusätzlich mit weiteren Molekülen, z.B. rekombinanten oder/und nativen Antigenen oder Antigengemischen, beschichtet sein. Die Ribonukleoproteine können direkt durch adsorptive oder kovalente Bindung auf der Festphhase immobilisiert werden oder indirekt über ein spezifisches Bindepaar, insbesondere Biotin/Streptavidin, wobei zunächst die Festhase mit einem Partner des spezifischen Bindepaares beschichtet wird und das mit dem zweiten Partner des spezifischen Bindepaares gekoppelte Ribonukleoprotein dann auf die vorbeschichtete Festphase aufgebracht wird. Der Nachweis kann auf herkömmliche Weise, beispielsweise unter Verwendung eines markierten Antikörpers, insbesondere eines Maus-Anti-human-IgG-POD (POD = Peroxidase) durchgeführt werden. Als Markierungsgruppen finden insbesondere Elektrochemilumineszenzmarkierungen, Fluoreszenzmarkierungen, Enzymmarkierungen, Sol-Partikel, wie z.B. Latexpartikel oder Goldpartikel und radioaktive Markierungen Verwendung.

Die Erfindung wird durch die Figuren und die Beispiele weiter erläutert, wobei
- Figur 1: die Aminosäuresequenz der rekombinanten Antigene SSA60 M4-C6 (obere Sequenz) und SSA60 M56 (untere Sequenz) darstellt;

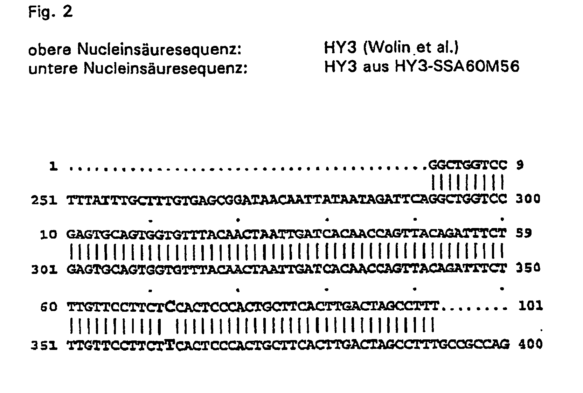

- Figur 2: einen Vergleich der von Wolin et al. in Proc. Natl. Acad. Sci. USA 81 (1984), 1996-2000 veröffentlichten Sequenz von HY3 (obere Sequenz) und der im Klon HY3-SSA60 M56 (untere Sequenz) verwendeten Gensequenz. Die Punktmutation ist durch einen dicker gedruckten Buchstaben gekennzeichnet;

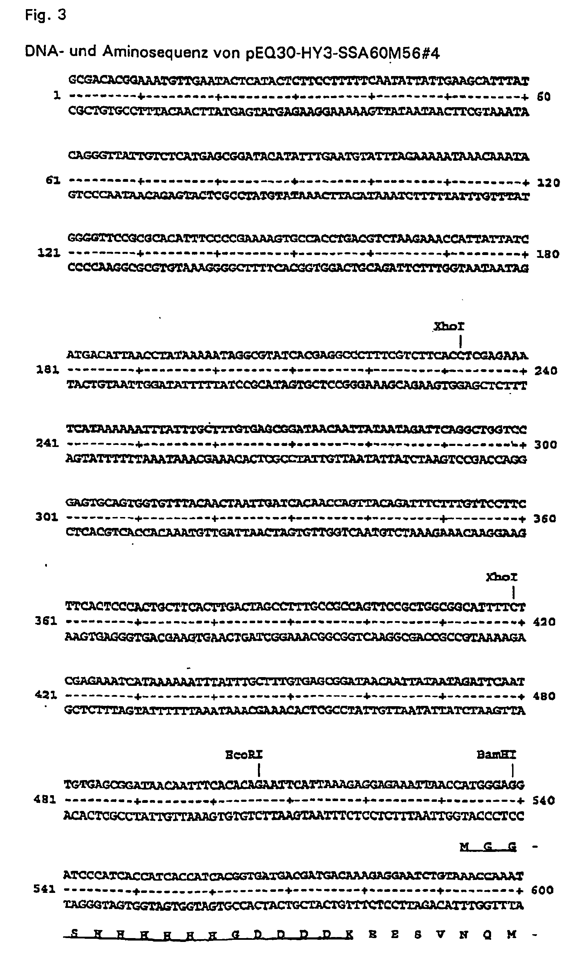

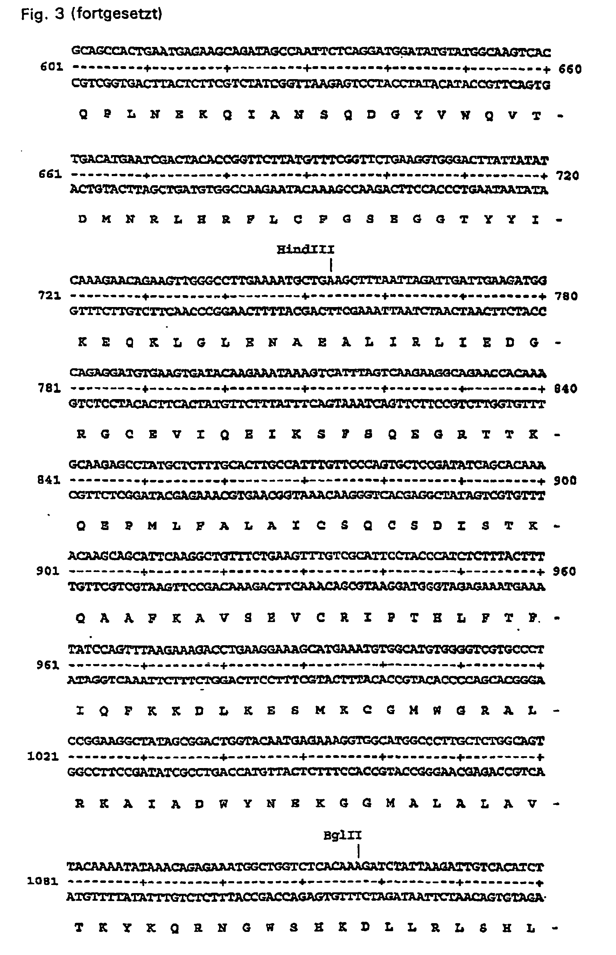

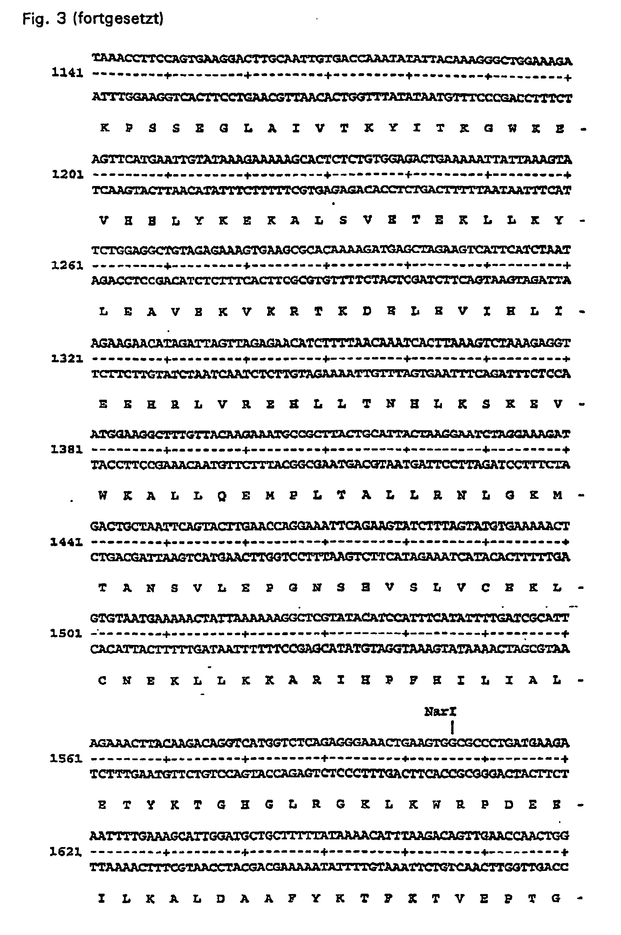

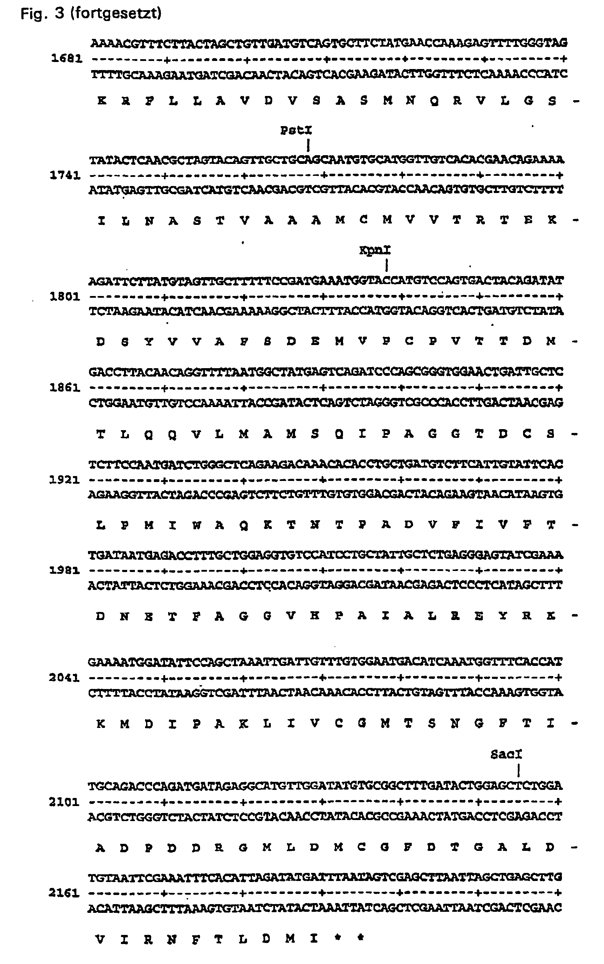

- Figur 3: die DNA- und Aminosäuresequenz von pQE30-HY3-SSA60 M56 #4 zeigt, wobei das HY3-Gen zwischen den zwei Xhol- Stellen (bp 232 - 419) angeordnet ist, während das SSA60 M56 stromab der EcoRI-Stelle (Translationsstart bei bp 533) zu finden ist. Charakteristische Restriktionsstellen sind angegeben. Die unterstrichenen Aminosäuren werden vom Vektor codiert, die anderen gehören zur humanen SSA60- Sequenz.

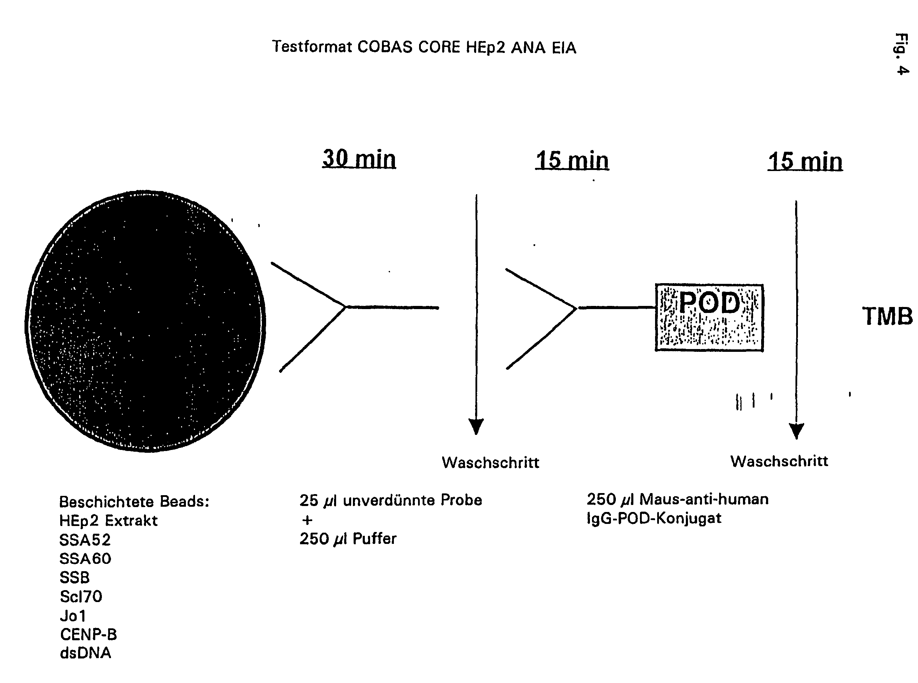

- Figur 4: zeigt ein Testformat mit beschichteten Beads, wobei die Beads neben den erfindungsgemäßen Ribonukleoproteinen weitere Erkennungsmoleküle tragen können, beispielsweise einen HEp2-Extrakt, SSA52, SSA60, SSB, Scl70, Jo1, CENP-B oder/und dsDNA. Die beschichteten Beats werden dann mit unverdünnter Probe, beispielsweise 25 µl, und einem Puffer, beispielsweise 250 µl, inkubiert, beispielsweise für 30 Minuten. Nach einem Waschschritt wird ein Nachweisreagenz, beispielsweise 250 ml Maus-Anti-Human-IgG-POD-Konjugat zugegeben und 15 Minuten inkubiert. Nach einem weiteren Waschschritt folgt der Nachweis mit TMB.

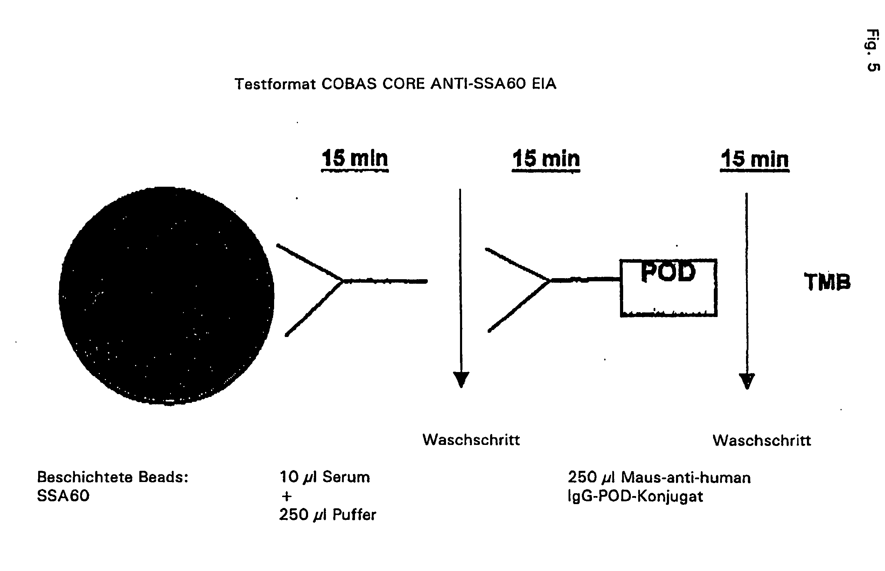

- Figur 5: zeigt ein Testformat mit Beads, die mit SSA60 beschichtet sind. Die beschichteten Beads werden mit 10 µl Serum als Probe und 250 µl Probepuffer für 15 min inkubiert. Nach einem Waschschritt wird 250 µl Maus-Anti-Human-IgG-POD- Konjugat zugegeben und 15 min inkubiert. Nach einem weiteren Waschschritt erfolgt die Auswertung mit TMB.

### Beispiel 1

### Herstellung eines rekombinanten HY3-SSA60-Antigens

Es wurde ein SSA60-M56 genanntes Antigen verwendet, welches ein modifiziertes rekombinantes SSA60-Protein mit Änderungen am N- und C-terminalen Ende ist, was die Möglichkeit eröffnet, ein rekombinantes Protein mit der Aminosäuresequenz des nativen SSA60-Proteins herzustellen, wobei keine Aminosäuren vom Vektor codiert werden, sowie ein rekombinantes SSA60-Antigen, welches nur am N-terminalen Ende eine abspaltbare Markierung aufweist.

HY3 codiert für eine kurze RNA, mit der das rekombinante SSA60-Protein assoziiert oder gebunden wird. Die Assoziierung von SSA60 mit HY3 induziert Konformationsepitope des Antigens, die zu einem unterschiedlichen immunologischen Verhalten im Vergleich zu einem SSA60-Protein führen, das keine HY3-RNA enthält. Die beiden Autoimmunantigene SSB und SSA60 binden an das gleiche RNA-Molekül, während SSA52 wahrscheinlich direkt mit dem SSA60-Protein assoziiert ist.

Bei dem hier verwendeten rekombinanten humanen SSA60-Protein handelt es sich um die Form b, die im Klon pQE30 HY3 SSA60 M56 translatiert wird. Sie codiert für insgesamt 553 Aminosäuren. Die ersten zwölf Aminosäuren am N-terminalen Ende des Proteins werden vom Expressionsvektor codiert. Diese abspaltbare Markierung enthält eine geladene Gruppe von Aminosäuren, welche die Löslichkeit des Antigens erhöht und eine Gruppe von sechs His für die Affinitätsreinigung über Ni-NTA.

Der in Figur 1 gezeigte Vergleich der Aminosäuresequenz der rekombinanten Antigene SSA60 M4-C6 und SSA60 M56 zeigt, daß das N-terminale Ende des rekombinanten SSA60-Antigens geändert wurde von MRGSHHHHHHGSMEES... (Sequenz eines bisher verwendeten Klons) zu MRGSHHHHHHGDDDDKEES...

In der neuen Sequenz SSA60 M56 wurde eine DDDK-Sequenz nach dem Abschnitt mit 6 His-Aminosäuren eingeführt, welche eine Spaltsequenz für Protease Bovine Enterokinase ist. Dies ermöglicht die Eliminierung des MRGSHHHHHHGDDDDK-Peptids nach Reinigung des Antigens über Ni-NTa. Das C-terminale Ende des rekombinanten Antigens wurde von ...IRNFTLDMIVD** verändert (bisher verwendeter Klon) zu ...IRNFTLDMI**. Die Sterne markieren repetitive Translationsstoppsignale am Ende des Proteins. Die Sequenz ... DMI stellt das C-terminale Ende des nativen und rekombinanten Antigens dar.

HY3 ist eine kleine RNA mit 101 Basen und hat eine definierte Sekundärstruktur. Die Bindung von SSA60 erfolgt an die Basis der festen Struktur. Im Vergleich zur im Stand der Technik veröffentlichten Struktur von HY3 (Wolin et al., PNAS USA 81 (1984), 1996-2000) weist die hier verwendete Sequenz einen Unterschied in der Schleife 2 der RNA auf (siehe Figur 2).

Die DNA-Sequenz des rekombinanten SSA60-Proteins und des HY3-RNA-Gens ist in Figur 3 gezeigt. Charakteristische Restriktionsstellen im Klon sind angezeigt. So ergibt eine Restriktionsspaltung der Plasmid-DNA des Klons pQE30 HY3 SSA60 M56, z.B. durch Xhol, BgIII und Sacl, vier DNA-Fragmente mit 187 bp, 698 bp, 1.038 bp und den Vektor (= 3.400 bp).

### Beispiel 2

### Herstellung des Expressionsvektors.

Es wurde der Expressionsvektor pQE30 verwendet, welcher ein kleines Plasmid mit 3.462 bp ist (erhältlich von Qiagen). Dieser Expressionsvektor wurde speziell für eine Expression von Proteinen in E.coli entworfen. Er enthält ein regulierbares Promotor/Operator-Element und eine starke Ribosombindestelle vor mehreren Klonierungsstellen (BamHI und HindIII neben anderen), die stromab einer Gruppe mit 6 His liegen. Die Expression des Gens unter Kontrolle des Promotor/Operator-Elements wird bei einer gegebenen Zelldichte durch Addition von IPTG induziert, welches den Repressor inaktiviert und den Promotor freisetzt.

Das rekombinante Plasmid pQE30 HY3 SSA60 M56, Klon 4, ergibt eine intrazelluläre Koexpression des rekombinanten humanen SSA60-Proteins und der HY3-RNA in E.coli. Zusammen bilden diese Komponenten einen Komplex mit Konformationsepitopen. Die Markierung mit 6 His erlaubt die Reinigung des Antigens durch eine Metallchelat-Affinitätschromatographie. Nicht denaturierende Reinigungsbedingungen sind erforderlich, um die Konformationsepitope des Komplexes zu erhalten.

Der lac-Repressor wird durch ein separates Plasmid codiert, das pREP4 genannt wird, welches mit dem Expressionsvektor pDS56 RBSII kompatibel ist. Das Plasmid pREP4 (von Qiagen) trägt einen Kanamycinresistenzfaktor, während der Expressionsvektor gegenüber dem Antibiotikum Ampicillin resistent ist.

Die Auswahl des gewünschten Klons pQE30 HY3 SSA60 M56, Klon 4, mit pREP4 wird durch Züchten in Gegenwart der zwei Antibiotika Ampicillin und Kanamycin durchgeführt.

Zur Herstellung des Klons pQE30 HY3 SSA60 M56 wurde zunächst die HY3-RNA in vitro durch PCR (Polymerasekettenreaktion) unter Verwendung der Primer HY3F und HY3R synthetisiert. Die Sequenz der Primer war wie folgt:
Primer HY3-SynF:
Primer HY3-SynR:

Fünf 100 µl PCR-Reaktionsgemische wurden hergestellt, die 10 *µl* 1 Ox Taq-Puffer (Pharmacia), 8 µl dNTP (1 mM dATP, dCTP, dGTP, dTTP), 3 µl von jedem der Primer HY3-SynF und HY3-SynR (jeweils 50 pmol/µl), 62 µl H₂O und 1 Tropfen Mineralöl (Sigma, M-3516) enthielten. Ein Heißstart wurde im ersten Zyklus bei 60°C mit 10 µl (5 Einheiten Pharmacia Taq und 2,5 Einheiten Pfu [Stratagene] in 1 xTaq-Puffer) initiiert. Die DNA-Amplifikation wurde in einem Perkin Elmer Cycler 9600 unter Verwendung des folgenden Programms durchgeführt:
1. Zyklus 1
   - 30 Sekunden bei 98°C
   - 2 Minuten bei 60°C
   - 30 Sekunden bei 72°C
2. Zyklen 2 bis 6
   - 30 Sekunden bei 95°C
   - 20 Sekunden bei 65°C
   - 30 Sekunden bei 72°C

Die PCR-Fragmente wurden gesammelt, mit den Restriktionsenzymen Xhol und Kpnl geschnitten, über Agarosegel gereinigt und in einen Bluescript II KS-Vektor kloniert, wobei das Gen HY3 für Transkriptionsstudien unter die Kontrolle des T7-Polymerasepromotors gestellt wurde. Dies ergab den Klon pBSII-HY3RNA3-1.

Die erwartete Sequenz des Gens HY3 wurde gefunden, mit Ausnahme einer Punktmutation, die in Figur 2 dargestellt ist. In vitro transkribierte HY3-RNA wurde für die Renaturierung von SSA60-Antigen verwendet, was eine erhöhte positive immunologische Reaktivität des Antigens ergab.

Um die Synthese eines rekombinanten SSA60-Antigens ohne zusätzliche Aminosäuren im Vergleich zur nativen Sequenz zu ermöglichen, wurden vektorcodierte Aminosäuren im SSA60-Klon, der für die Koexpression von SSA60 und HY3 verwendet wurde, eliminiert. Das N-terminale Ende des rekombinanten SSA60-Antigens wurde dafür verändert auf MRGSHHHHHHGDDDDKEES... (Klon SSA60-M56). Das SSA60-Protein beginnt dabei mit den letzten drei Aminosäuren dieser Sequenz, nämlich EES..., während die restlichen Aminosäuren darstellen, die durch den Vektor codiert sind. Die Sequenzmodifikation wurde durch eine PCR-Reaktion eines 260 bp-Fragments erhalten, das die Basenpaare 506 - 758 der in Figur 3 gezeigten SSA60-M56-Sequenz abdeckt. Die zwei Primer SSA60 M6-NF und SSA60 M6-NRev hatten die folgende Sequenz:
1. SSA60 M6-NF
2. SSA60 M6-NRev
   5' ctaattaaagcttcagcattttcaagg 3'

Die amplifizierte DNA von 260 bp wurde geschnitten und ersetzte das EcoRI/HindIII-Fragment des Klons SSA60-M4-C6. Die Sequenz des neuen Klons ist in Figur 3 gezeigt.

Das C-terminale Ende des rekombinanten Antigens wurde von ...IRNFTLDMIVD** (Klon SSA60 M4-C6) in ...IRNFTLDMI** (Klon SSA60-M56) modifiziert. Die Sequenzmodifikation wurde durch eine PCR-Reaktion eines 260 bp-Fragments erhalten, das die bp 506 - 758 der in Figur 3 gezeigten SSA60-M56-Sequenz umfaßt. Die zwei Primer SSA60M5-CF und SSA60M5-CRev hatten die folgende Sequenz:
1. SSA60M5-CF
2. SSA60M5-CRev
   5' aggcagctctagagcggcggatttgtcc 3'

Die amplifizierte DNA von 1.200 bp wurde mit den Restriktionsenzymen Sacl und Xbal geschnitten und das gereinigte Fragment in SSA60M4-C6 insertiert, was das zuvor vorliegende Fragment Sacl-Xbal ersetzte (in Figur 3 ist der Ort des Fragments stromab von Sacl bei bp 2.155 gezeigt; Xbal ist nicht gezeigt, da es im Vektor pQE lokalisiert ist).

Der durch diese Veränderungen erhaltene Klon wurde pQE30-SSA60-M56 #6 genannt. Um SSA60 von M56SSA60M4-Cl zu unterscheiden, wurde die Restriktionsstelle für Sall am Ende des SSA60-Antigens entfernt.

Es wurde ein Klon synthetisiert, der HY3 und SSA60 in E.coli koexprimiert und pQE30-HY3-SSA60M56 #4 genannt wurde. Für diese Zwecke wurde HY3 vor das SSA60-Gen in den Klon pQE30 SSA60 M56 #6 kloniert, wobei das Gen HY3 unter die Kontrolle der identischen Promotor- und Operatorsequenzen wie das SSA60-Gen gestellt wurde.

Das HY3-Gen wurde zur Insertion in die Xhol-Stelle von pQE30 durch Verändern seiner Sequenz, wie oben beschrieben, mit der Hilfe des Primers HYFPQE und HYRPQE durch PCR-Modifizierung vorbereitet. Die Sequenzen der zwei Primer waren:
HYFPQE
HYRPQE

Das Produkt dieser Reaktion wurde mit dem Restriktionsenzym Xhol geschnitten und in die Xhol-Stelle des Klons pQE30 SSA60 M56 #6 kloniert, was den neuen Klon pQE30-HY3-SSA60M56 #4 ergab. Die DNA-und Proteinsequenz des neuen Klons sind in Figur 3 gezeigt.

### Beispiel 3

### Expression von pQE30-HY3-SSA60M56, Klon 4

Der für eine Überexpression des rekombinanten Ribonukleoprotein-Komplexes HY3-SSA60 verwendete E.coli-Stamm ist ein XL1 Blue (Stratagene) genanntes E.coli C-Derivat. Für die Überexpression des rekombinanten HY3-SSA60-Komplexes wird eine Kultur von pQE30-HY3-SSA60M56 #4 in XL1 mit pREP4 bei 28°C in einem selektiven TB-Medium (enthaltend Ampicillin und Kanamycin) gezüchtet. Bei etwa 25 % der maximalen Zelldichte wird eine Transkription und Translation des rekombinanten Proteins mit 1 mM IPTG induziert und das Wachsen für 3 bis 5 Stunden bei 28°C fortgesetzt. Bakterienpellets, die das rekombinante Protein enthalten, werden durch Zentrifugation gesammelt und das Zellpellet bis zur Reinigung des überexprimierten rekombinanten Antigens gefroren gelagert.

Eine Optimierung der Expression von immunologisch reaktivem Material zeigte, daß insbesondere die Kombination von 1.) einer Fermentation bei geringer Temperatur und 2.) nicht denaturierende Reingungsprotokolle zufriedenstellende Ergebnisse ergaben.

Zur Kontrolle der Überexpression des HY3-SSA60-Antigens wurden Proteinaliquots der induzierten Bakterien nach Elektrophorese auf einem SDS-Polyacrylamidge,1 angefärbt. Das rekombinante HY3-SSA60-Antigen wandert als Hauptproteinbande bei 75 kD, verglichen zu vorgefärbtem Seeblue-Marker (Novex # LC 5625).

### Beispiel 4

### Native Reinigung des koexprimierten HY3-RNA-SSA60-Konstrukts

Als Extraktionspuffer wurde ein Gemisch von 1 mM PMSF (hergestellt aus 0,2 M PMSF in 2-Propanol), Aprotinin (10 mg/ml in Wasser) in einer Menge von 10 µg/g Biomasse, Leupeptin (1 mg/ml in Wasser) in einer Menge von 15 µg/g Biomasse, Pepstatin (1 mg/ml in Methanol) in einer Menge von 15 µg/g Biomasse und Lysozym (10 mg/ml im Lysepuffer) in einer Menge von 100 µl/g Biomasse verwendet. Der Puffer wurde bei Raumtemperatur zugegeben, wobei ein Extraktionsverhältnis von 1 g Biomasse/5 ml Puffer umfassend 10 mM Tris, pH 7,0, 500 mM NaCl, 10 % Glycerin und 0,2 % Tween 20 eingehalten wurde. Zur Extraktion wurde die Suspension in Eiswasser für 10 Minuten mit 5 Sekunden Intervallen ultraschallbehandelt. Die Abzentrifugierung erfolgte bei 4°C für 10 Minuten bei 10.000 g. Das gewünschte Ribonukleoprotein HY3-RNA-SSA60 liegt im Überstand in Lösung vor. Der Überstand wurde mit SDS-PAGE und Western Blot behandelt. Anschließend erfolgte eine Nickelreinigung der positiven Überstände unter nativen Laufbedingungen unter Verwendung eines Säulenlaufpuffers, der 10 mM TRIS, pH 7,0, 500 mM NaCl, 10 % Glycerin und 0,2 % Tween 20 umfaßte. Die Elution erfolgte mit 5 mM, 10 mM, 20 mM, 50 mM bzw. 100 mM Imidazol im Säulenlaufpuffer. Die Elutionsfraktionen wurden mittels SDS-PAGE und Western Blot analysiert und die das gewünschte Ribonukleoprotein enthaltendenen Fraktionen gesammelt.

### Beispiel 5

### Verwendung des HY3-RNA-SSA60-Ribonukleoproteins in einem diagnostischen Verfahren

Das für das SSA60-Vergleichsexperiment herangezogene Cobas Core Anti-SSA60 EIA Testformat ist in Figur 5 gezeigt. Es ist vergleichbar mit dem Testformat für den Cobas Core HEp2 ANA EIA (Kombi-Test, vgl. Fig. 4), jedoch ist lediglich SSA60-Antigen zur Beschichtung verwendet worden.

### 1) Beschichtung

SSA60-Antigene wurden in Phosphatpuffer mit 0,15 m NaCl (PBS) und 2 mM EDTA bei Raumtemperatur (RT) für 14 bis 16 h mit gamma-bestrahtlen Polystyrolkugeln (NUNC, Dänemark) bis zur Sättigung (max. SSA60-Bindungskapazität) inkubiert.

Nach 3-maligem Waschen der Kugeln mit PBS wurden diese in 2 % BSA/PBS-Lösung zur Abstättigung freier Bindungsstellen überführt (2-3 h bei Raumtemperatur (RT)). Die SSA60-Kugeln wurden danach für 2 h bei RT unter Vakuum getrocknet, in spezielle Trockenbehälter (Cobas Core) überführt und bis zum Gebrauch bei 4°C gelagert.

### 2) Testführung (EIA automatisch durch Cobas Core durchgeführt)

10 µl Serumprobe plus 250 µl Verdünnungsmittel plus SSA60-Kugel werden 15 min bei 37°C inkubiert. Danach wird dreimal mit entnionisiertem Wasser gewaschen mit 250 µl Maus-anti-Human-IgG-Peroxidase 15 min bei 37°C inkubiert. Nach erneutem dreimaligem Waschen mit entionisiertem Wasser und mit Peroxidasesubstrat TMB 15 min bei 37°C inkubiert. Die Auswertung erfolgt durch Messung der OD bei 650 nm. Das Farbsignal bei OD650 ist proportional zur spezifischen Antikörperkonzentration im Serum.

### 3) Seren

Die getesteten Seren sind zum Teil kommerzieller (Serum A: BM69660, Serum B: BM6020, Serum C: BM 7529, Serum D: BM69440, Serum E: M48/4713), zum Teil klinischer (Serum F: SS12) Herkunft. Bei letzterem handelt es sich um ein sehr selten vorkommendes Serum von einem Patienten mit Sjögren's Syndrom. Die Reaktivität von SSA60-Antikörpern wurde mit verschiedenen kommerziell erhältlichen Tests (ELISA) nachgewiesen. Die verwendet kommerziellen Tests beinhalten dabei entweder natives oder rekombinantes SSA60 Antigen. Alle Positivseren waren mit dem beschichteten nativen SSA60-Antigen von Immunovision positiv detektierbar, während ein Teil dieser Proben mit dem beschichteten reinen SSA60-Protein aus E.coli (denaturiert) ein negatives Testresultat lieferten. Letztere Proben konvertierten durch den Einsatz von nativ gereinigtem hY3-RNA-SSA60 anstatt denaturiertem SSA60 von negativ zu positiv.

Damit wurde die gewünschte Reaktivität des hY3-RNA-SSA60-Antigens im Test bewiesen.

**Tabelle 1**

| Versuch | In Sättigung beschichtetes SSA60-Antigen | | | | | |
|---|---|---|---|---|---|---|
| Positivseren | OD650 nm | OD650nm | OD650nm | OD660/Cutoff | OD650/Cutoff | OD650/Cutoff |
| | SSA60 | Immunovision | SSA60-hY3 | SSA60 | Immunovision | SSA60-hY3 |
| Serum A | 3,50 | 3.50 | 3,30 | 5,83 | 38,89 | 7,49 |
| 1:10 verd. | 0,98 | 3,50 | 1,41 | 1,63 | 38,89 | 3,20 |
| Serum B | 2,55 | 3,50 | 3,50 | 4,24 | 38,89 | 7,85 |
| 1:10 verd. | 0,46 | 2,63 | 1,15 | 0,77 | 29,22 | 2,62 |
| Serum C | 3,38 | 3,50 | 2,78 | 5,63 | 38,89 | 6,32 |
| Serum D | 3,50 | 3,50 | 3,44 | 5,83 | 38,89 | 7,83 |
| 1:10 verd. | 0,44 | 3,50 | 1,67 | 0,73 | 38,89 | 3,80 |
| Serum E | 0,50 | 3,50 | 3,46 | 0,84 | 38,89 | 7,87 |
| 1:10 verd. | 0,28 | 3,50 | 1,63 | 0,44 | 38,89 | 3,71 |
| Serum F | 0,24 | 3,50 | 1,22 | 0,40 | 38,89 | 2,77 |

| Blutspenderseren | OD650 nm | OD650 nm | OD650 nm | OD650/Cutoff | OD650/Cutoff | OD650/Cutoff |
|---|---|---|---|---|---|---|
| | SSA60 | Immunovision | SSA60-hY3 | SSA60 | Immunovision | SSA60-hY3 |
| 1 | 0.14 | 0.05 | 0.07 | 0.24 | 0.54 | 0.17 |
| 2 | 0.10 | 0.14 | 0.17 | 0.16 | 1.56 | 0.38 |
| 3 | 0.13 | 0.06 | 0.05 | 0.22 | 0.63 | 0.11 |
| 4 | 0.35 | 0.06 | 0.14 | 0.59 | 0.67 | 0.33 |
| 5 | 0.23 | 0.06 | 0.09 | 0.38 | 0.66 | 0.20 |
| 6 | 0.07 | 0.06 | 0.06 | 0.12 | 0.67 | 0.15 |
| 7 | 0.05 | 0.05 | 0.05 | 0.08 | 0.60 | 0.11 |
| 8 | 0.04 | 0.05 | 0.02 | 0.07 | 0.50 | 0.04 |
| 9 | 0.06 | 0.05 | 0.28 | 0.10 | 0.51 | 0.63 |
| 10 | 0.12 | 0.05 | 0.20 | 0.19 | 0.61 | 0.45 |
| 11 | 0.08 | 0.06 | 0.15 | 0.13 | 0.69 | 0.34 |
| 12 | 1.11 | 0.07 | 0.10 | 1.85 | 0.76 | 0.23 |
| 13 | 0.62 | 0.08 | 0.09 | 1.04 | 0.86 | 0.20 |
| 14 | 0.30 | 0.08 | 0.14 | 0.49 | 0.90 | 0.32 |
| 15 | 0.07 | 0.13 | 0.04 | 0.12 | 1.44 | 0.10 |
| 16 | 0.21 | 0.06 | 0.07 | 0.35 | 0.62 | 0.15 |
| 17 | 0.20 | 0.05 | 0.08 | 0.34 | 0.59 | 0.18 |

| Blutspender | OD660 nm | OD650 nm | OD650 nm | OD650/Cutoff | OD650/Cutoff | OD650/Cutoff |
|---|---|---|---|---|---|---|
| | SSA60 | Immunovision | SSA60-hY3 | SSA60 | Immunovision | SSA60-hY3 |
| MW1 | 0.25 | 0.07 | 0.17 | | | |
| STA1 | 0.27 | 0.03 | 0.21 | | | |
| MWY1 +1,5xSTA1 | 0.66 | 0.13 | 0.59 | | | |
| MW2 | 0.17 | 0.06 | 0.13 | | | |
| STA2 | 0.14 | 0.01 | 0.10 | | | |
| Cutoff ( > MW2 + 3xSTD2) | 0.6 | 0.09 | 0.44 | | | |

Tabelle 1 zeigt die Ergebnisse für Positivseren und Negativseren (Blutspenderseren), die unter Verwendung von rekombinant in E.coli ohne RNA hergestelltem SSA60, SSA60 von Immunovision (natives SSA60 aus Rindermilz) und erfindungsgemäß hergestelltem SSA60-hY3 erhalten werden. Wie hieraus zu ersehen ist, liegt die Sensitivität des erfindungsgemäß hergestellten SSA60-hY3 deutlich über der des SSA60 ohne RNA. Eine Probe wurde als negativ eingestuft, wenn OD650 nm/Cutoff < 1 und als positiv eingestuft, wenn OD650 nm/Cutoff > 1. Wie der Tabelle zu entnehmen ist, werden die Positivseren B (1:10 verdünnt), D (1:10 verdünnt), E (unverdünnt und 1:10 verdünnt) sowie die klinische Probe Serum F, die mit SSA60 ohne RNA als negativ eingestuft werden, mit dem erfindungsgemäß hergestellten SSA60-hY3 richtigerweise als positiv erkannt.

Darüber hinaus weist das erfindungsgemäß hergestellte SSA60-hY3 auch eine höhere Spezifität als SSA60 ohne RNA oder SSA60 von Immunovision auf, wie dem zweiten Teil der Tabelle 1 entnommen werden kann. Dort sind die Ergebnisse von 17 Blutspendern (Negativseren) aufgeführt. Während hier mit SSA60 und auch mit SSA60 von Immunovision noch zwei Negativseren (Nr. 12 und 13, bzw. Nr. 2 und 15) als positiv eingestuft wurden, werden mit dem erfindungsgemäß hergestellten SSA60-hY3 alle Seren korrekt als Negativ erkannt.

## Patentansprüche

1. Verfahren zur rekombinanten Herstellung eines SSA60-Ribonukleoproteins oder eines Derivats davon umfassend die Schritte:
(a) Bereitstellen einer prokaryontischen Wirtszelle, die (i) mindestens eine für eine Ribonukleinsäurekomponente des SSA60-Ribonukleoproteins kodierende DNA enthält und (ii) mindestens eine für eine Proteinkomponente des SSA60-Ribonukleoproteins kodierende DNA enthält,
(b) Exprimieren der DNAs (i) und (ii) unter Bedingungen, bei denen ein Ribonukleoprotein gebildet wird und
(c) Gewinnen des Ribonukleoproteins,
wobei ein Derivat gegenüber der nativen Form in der Sequenz der Ribonukleinsäure und/oder der Sequenz des Proteins modifiziert ist, die Fähigkeit der Komponenten zur Assoziierung zum Ribonukleoprotein erhalten bleibt.

2. Verfahren nach Anspruch 1,
**dadurch gekennzeichnet,**
**dass** es sich bei der Ribonukleinsäurekomponente um eine HY1-, HY3-, HY4- oder HY5-RNA handelt.

3. Nukleinsäurekonstrukt umfassend einen Abschnitt, der eine für eine SSA60-Proteinkomponente kodierende DNA enthält und einen Abschnitt, der eine für eine Ribonukleinsäurekomponente des SSA60-Ribonukleoproteins kodierende DNA enthält.

4. Konstrukt nach Anspruch 3,
**dadurch gekennzeichnet,**
**dass** es sich bei der Ribonukleinsäurekomponente um eine HY1-, HY3-,HY4- oder HY5-RNA handelt.

5. Konstrukt nach einem der Ansprüche 3 und 4,
**dadurch gekennzeichnet,**
**dass** es eine gleichzeitige Induktion der Ribonukleinsäurekomponente und der Proteinkomponente ermöglicht.

6. Konstrukt nach einem der Ansprüche 3 bis 5,
**dadurch gekennzeichnet,**
**dass** die kodierenden Bereiche jeweils operativ mit einem lac-Operator verknüpft sind.

7. Rekombinante prokaryontische Zelle,
**dadurch gekennzeichnet,**
**dass** sie (i) mindestens eine für eine Ribonukleinsäurekomponente eines SSA60-Ribonukleoproteins kodierende DNA und (ii) mindestens eine für eine Proteinkomponente des SSA60-Ribonukleoproteins kodierende DNA enthält.

8. Rekombinantes Ribonukleoprotein erhältlich mit einem Verfahren nach einem der Ansprüche 1 und 2,
**dadurch gekennzeichnet,**
**dass** ein SSA60-Ribonukleoprotein mit der Ribonukleinsäurekomponente HY3-, HY4- oder HY5-RNA exprimiert wird.

9. SSA60-Protein mit der in Figur 1 gezeigten Sequenz SSA60M56 gegebenenfalls in Assoziation mit RNA.

10. Verwendung eines Ribonukleoproteins nach Anspruch 8 oder 9 für in vitro diagnostische Verfahren.

11. Verwendung nach Anspruch 10,
**dadurch gekennzeichnet,**
**dass** ein rekombinantes SSA60-Ribonukleoprotein für die Diagnostik von Autoimmunerkrankungen eingesetzt wird.

12. Verfahren zum Nachweis eines Analyten in einer Probe unter Verwendung eines Analyt-spezifischen Rezeptors,
**dadurch gekennzeichnet,**
**dass** man als Rezeptor ein Ribonukleoprotein nach Anspruch 8 oder 9 einsetzt.

## Claims

1. Process for the recombinant production of an SSA60 ribonucleoprotein or a derivative thereof which comprises the steps:
(a) preparing a prokaryotic host cell which contains (i) at least one DNA coding for a ribonucleic acid component of the SSA60 ribonucleoprotein and (ii) at least one DNA coding for a protein component of the SSA60 ribonucleoprotein,
(b) expressing the DNAs (i) and (ii) under such conditions that a ribonucleoprotein is formed and
(c) isolating the ribonucleoprotein,
wherein a derivative has a modified sequence of the ribonucleic acid and/or of the protein compared to the native form, where the ability of the components to associate to form the ribonucleoprotein is retained.

2. Process according to claim 1,
**characterized in that**
the ribonucleic acid component is a HY1, HY3, HY4 or HY5 RNA.

3. Nucleic acid construct comprising a section which contains a DNA coding for an SSA60 protein component and a section containing a DNA coding for a ribonucleic acid component of the SSA60 ribonucleoprotein.

4. Construct according to claim 3,
**characterized in that**
the ribonucleic acid component is a HY1, HY3, HY4 or HY5 RNA.

5. Construct according to one of the claims 3 and 4,
**characterized in that**
it enables a simultaneous induction of the ribonucleic acid component and the protein component.

6. Construct according to one of the claims 3 to 5,
**characterized in that**
the coding regions are each linked operatively with a lac operator.

7. Recombinant prokaryotic cell,
**characterized in that**
it contains (i) at least one DNA coding for a ribonucleic acid component of an SSA60 ribonucleoprotein and (ii) at least one DNA coding for a protein component of the SSA60 ribonucleoprotein.

8. Recombinant ribonucleoprotein obtainable using a process according to one of the claims 1 and 2,
**characterized in that**
an SSA60 ribonucleoprotein with the ribonucleic acid component HY3, HY4 or HY5 RNA is expressed.

9. SSA60 protein having the sequence SSA60M56 shown in figure 1 optionally associated with RNA.

10. Use of a ribonucleoprotein according to claim 8 or 9 for in vitro diagnostic methods.

11. Use according to claim 10,
**characterized in that**
a recombinant SSA60 ribonucleoprotein is used to diagnose autoimmune diseases.

12. Method for the detection of an analyte in a sample using an analyte-specific receptor,
**characterized in that**
a ribonucleoprotein according to claim 8 or 9 is used as the receptor.

## Revendications

1. Procédé de production recombinante d'une ribonucléoprotéine SSA60 ou d'un dérivé de celle-ci comprenant les étapes de :
(a) mise à disposition d'une cellule hôte procaryote qui (i) contient au moins un ADN codant pour un composant d'acide ribonucléique de la ribonucléoprotéine SSA60 et (ii) qui contient au moins un ADN codant pour un composant protéique de la ribonucléoprotéine SSA60,
(b) expression des ADN (i) et (ii) dans des conditions dans lesquelles une ribonucléoprotéine est formée, et
(c) obtention d'une ribonucléoprotéine,
dans lequel un dérivé est modifié par rapport à la forme native dans la séquence de l'acide ribonucléique et/ou la séquence de la protéine, la capacité des composants à s'associer à la ribonucléoprotéine étant conservée.

2. Procédé selon la revendication 1,
**caractérisé en ce que**
le composant d'acide ribonucléique est un ARN de HY1, HY3, HY4 ou HY5.

3. Construction d'acide nucléique comprenant un segment qui contient un ADN codant pour un composant de protéine SSA60 et un segment qui contient un ADN codant pour un composant d'acide ribonucléique de la ribonucléoprotéine SSA60.

4. Construction selon la revendication 3,
**caractérisée en ce**
**que** le composant d'acide ribonucléique est un ARN de HY1, HY3, HY4 ou HY5.

5. Construction selon l'une des revendications 3 et 4,
**caractérisée en ce qu'**elle permet une induction concomitante du composant d'acide ribonucléique et du composant protéique.

6. Construction selon l'une des revendications 3 à 5,
**caractérisée en ce**
**que** les domaines codants sont respectivement reliés fonctionnellement avec un opérateur lac.

7. Cellule procaryote recombinante,
**caractérisée en ce**
**qu'**elle contient (i) au moins un ADN codant pour un composant d'acide ribonucléique d'une ribonucléoprotéine SSA60 et (ii) au moins un ADN codant pour un composant protéique de la ribonucléoprotéine SSA60.

8. Ribonucléoprotéine recombinante pouvant être obtenue par un procédé selon l'une des revendications 1 et 2,
**caractérisée en ce**
**qu'**une ribonucléoprotéine SSA60 est exprimée avec l'ARN de HY3, HY4 ou HYS du composant d'acide ribonucléique.

9. Protéine SSA60 comportant la séquence SSA60M56 présentée sur la figure 1 éventuellement en association avec un ARN.

10. Utilisation d'une ribonucléoprotéine selon la revendication 8 ou 9, pour un procédé diagnostique *in vitro.*

11. Utilisation selon la revendication 10,
**caractérisée en ce**
**que** l'on utilise une ribonucléoprotéine SSA60 recombinante pour le diagnostic de maladies auto-immunes.

12. Procédé de détection d'un analyte dans un échantillon utilisant un récepteur spécifique à un analyte,
**caractérisé en ce**
**que** l'on utilise comme récepteur, une ribonucléoprotéine selon la revendication 8 ou 9.
